# EUROPEAN PATENT SPECIFICATION

(11) **EP 2 918 196 B1**
(45) Date of publication and mention of the grant of the patent: **30.11.2016**
(21) Application number: 14189737.1
(22) Date of filing: 21.10.2014
(51) Int. Cl.: A47C 7/38

(54) **Air-activated heated travel pillow**
Luftaktiviert beheiztes Reisekissen
Oreiller de voyage chauffant activé à air

(30) Priority: 11.03.2014 US 201461951182 P; 07.10.2014 US 201414508628
(43) Date of publication of application: 16.09.2015
(73) Proprietor: E&B Giftware LLC, Elmsford, NY 10523 (US)
(72) Inventor: Ross, Paul Howard, Pound Ridge, NY New York 10576 (US); Rubenstein, Scott, Ridgewood, NJ New Jersey 07450 (US)
(74) Representative: Beck Greener

(56) References cited:
- WO-A2-2011/122769
- CA-A1- 2 525 957
- US-A- 4 858 259
- US-A1- 2005 101 888
- US-A1- 2006 057 917
- US-A1- 2009 299 442

## Description

The present invention relates generally to travel and comfort items, and more particularly to a travel pillow comprising a heat pack.

Disposable air-activated heat packs safe for air travel, have not been implemented into a travel neck pillow to provide air travelers with soothing heat. Other methods of heat, such as *via* USB power, battery power, electric power, silica gel, instant chemical reactions (*e.g.,* MREs and flameless heaters), microwavable materials, hot water bottles, *etc.,* are inefficient, require heavy power consumption, are infeasible, and/or are not allowed on various forms of transportation, such as airplanes, due to hazardous or dangerous ingredients. Therefore, there is a need to be able to provide users with heating capable pillows that are both safe and efficient.

US 4858259 discloses a U-shaped pillow comprising a pocket comprising a heat pack.

The present invention provides for a pillow (100) comprising:
- a U-shaped pillow body comprising two legs (110, 112) and a base (114) therebetween, configured to receive and support a neck of a user;
- a pocket (120) formed on an inner radial portion of the U-shaped pillow body, wherein the inner radial portion is a portion at least partially adjacent to the back of the neck of the user when in use, wherein the pocket (120) is comprised of a lightweight, permeable, breathable, flexible, stretchable material, and is adjacent to the back of the neck of the user when in use; and
- a heat pack (130) contained in the pocket for a sustained, targeted, and controlled heat transfer to the user, wherein the heat pack is an air-activated, disposable heat pack comprising a soft, lightweight, air-permeable pouch.

Preferred features are set out in the dependent claims.

In a preferred embodiment, the U-shaped pillow body comprises one or more of foam, visco-elastic memory foam, polyurethane foam, cotton, polyester, polyester plush, spandex, micro-beads, and Polystyrene foam beads.

In a preferred embodiment of the pillow the pocket may be comprised of one or more of spandex, cotton, and polyester.

The pocket may have one or more open ends for removably receiving and containing the heat pack.

The pocket may further comprise a fastener at the one or more open ends for securing the heat pack therein, the fastener comprising one or more of a flap, spandex, Velcro®, a snap, a button, and a zipper.

The pocket may be configured to simultaneously and removably receive and contain a plurality of heat packs.

According to the invention, the heat pack of the pillow is an air-activated, disposable heat pack. The heat pack may be comprised of one or more of iron powder, sawdust, activated carbon, salt, water, and vermiculite to generate heat *via* an oxidation reaction.

By the heat pack in the pocket a user is provided with soothing, targeted, and controlled heat in a consistent, convenient, and comfortable application.

Heat packs, also known as body warmers, heat pads, warmers, heating pads, heating packs, hand warmers, foot warmers, toe warmers, pack heat, oxygen-activated heat packs, and/or air-activated chemical warming devices, are known to aid users in keeping parts of the body warm in cold weather environments as well as in heat therapy applications. Depending upon the chemical composition of the heat pack, a sustained, controlled heat can be generated for a period of up to 18 hours at a maximum temperature of 131-170 degrees Fahrenheit (55-77 degrees Celsius). A heat pack may be disposable. A heat pack may be safe for air travel. This feature allows preferred embodiments of the present invention to meet recent increased security measures for air travel, for example.

According to the invention the disposable heat pack contained in the pocket of the pillow comprises a soft, lightweight, air-permeable pouch. In one preferred embodiment, one side of the heat pack is air-permeable to more efficiently provide and optimally direct the targeted heat to a user.

The contents of the heat pack may include, but are not limited to, a mixture of iron powder, sawdust, activated carbon, salt, and water. Other ingredients may include cellulose and vermiculite. A summary of exemplary ingredients and their role in the chemical reaction is provided below:
Iron Powder- Iron is an active ingredient in the heat pack. When the iron combines with oxygen and the other ingredients in the pack, it starts to rust at an accelerated rate. The rusting process is what gives off the heat that warms the heat pack.
Sawdust - Sawdust is used to retain and release moisture in the heat pack, which in turns aids in the dispersion of the heat from the oxidation reaction.
Activated Carbon - Activated carbon may be added to heat packs because it helps absorb the oxygen. It also prevents external odors because unpleasant smelling chemicals released during the oxidation process stick to it.
Water - Water adds to the reaction within the heat pack as it helps disperse the created heat.
Salt - Salt may be used to accelerate the oxidation process, causing the iron to rust more quickly and generate more heat.
Optional Ingredients - Vermiculite may be added and used as an insulator; it helps the heat pack retain its heat. Vermiculite also absorbs excess moisture. Cellulose may be added as a filler. It holds and releases moisture as well.

In one preferred embodiment of the pillow, the heat pack uses a specific formulation of iron powder, water, salt, activated carbon, and vermiculite filled to a predetermined maximum weight given their size to provide a quick ramp-up temperature in a comfort temperature zone (55-63 degrees Celsius), and to also provide maintenance of this temperature once reached through an elapsed time of eight hours after initial activation. This composition, based on proportion, size, weight, and heat pack materials, provides a steady heat once the temperature is obtained.

When the heat pack is removed from airtight packaging and exposed to the air, an exothermic oxidation reaction activates, generating controlled and sustained heat from the pouch. Once the iron powder within the heat pack completely oxidizes and the heat pack cools, the pouch will no longer generate heat. For this reason, the heat pack is single use and disposable.

The foregoing and other aspects of the present invention are best understood from the following detailed description when read in connection with the accompanying drawings. For the purpose of illustrating the invention, there is shown in the drawings an embodiment that is presently preferred, it being understood, however, that the invention is not limited to the specific instrumentalities disclosed. Included in the drawings are the following Figures:

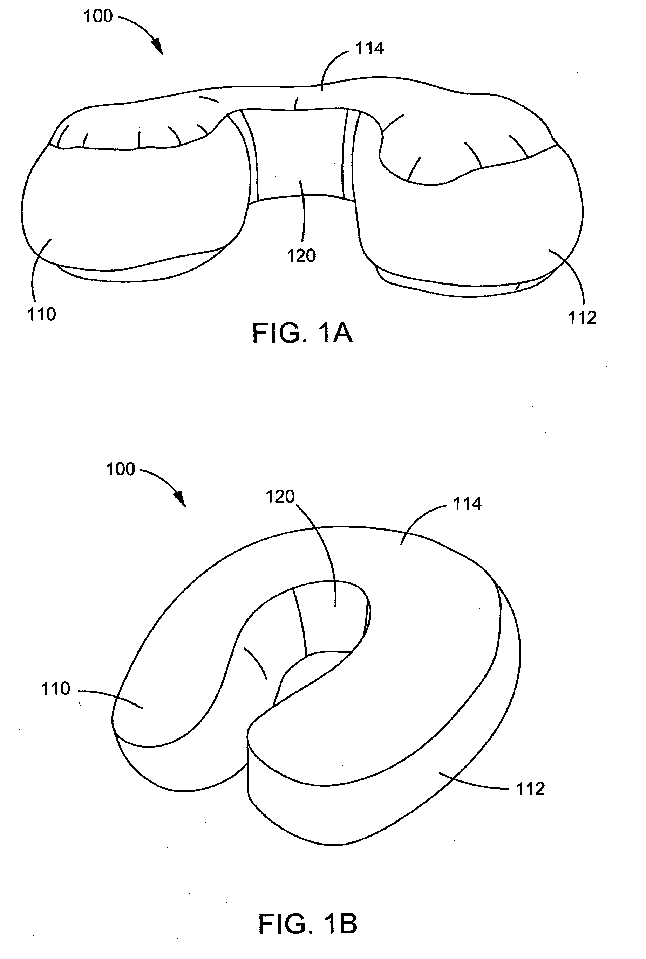

FIGs. 1A-1E illustrate a U-shaped travel neck pillow, according to the invention.

FIGs. 1A-1E illustrate a U-shaped travel neck pillow 100, according to an embodiment of the invention.

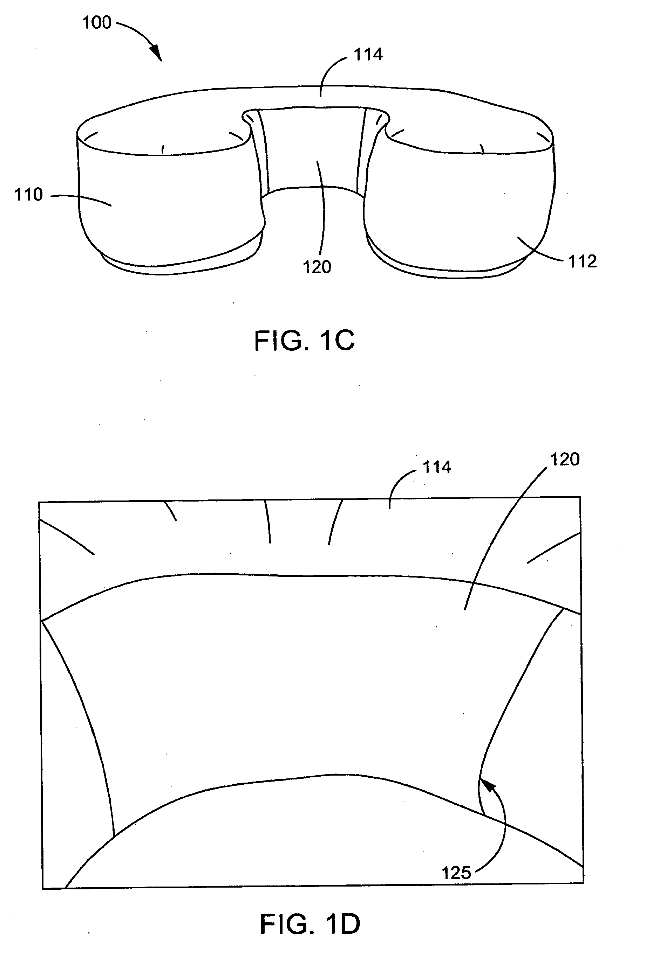

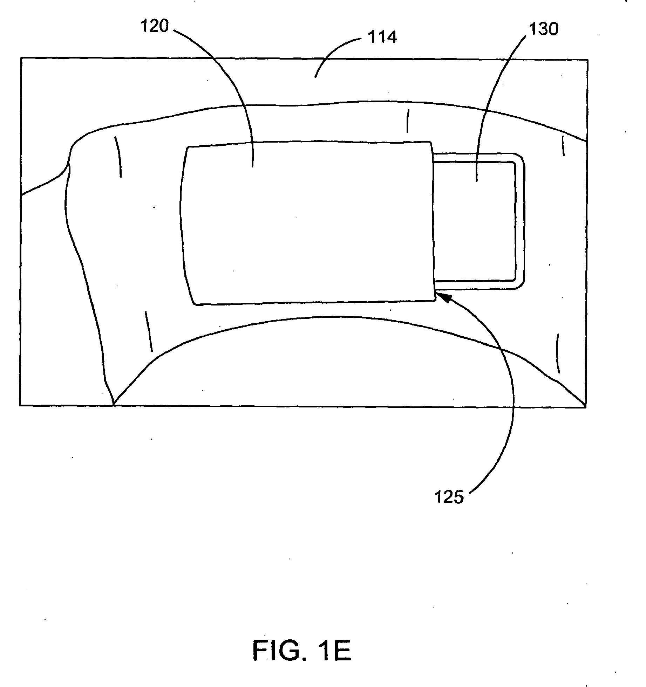

In the embodiment, as shown in FIGs. 1A, 1B, and 1C, a heated travel neck pillow 100 comprises a U-shaped pillow having with two leg portions 110, 112 and a base 114 therebetween, forming a U-shaped pillow body configured to receive and support a neck of a user. The heated travel neck pillow 100 also includes one or more pockets 120 formed on an inner radial portion of the U-shaped pillow body, where the inner radial portion is a portion at least partially adjacent to the neck of the user when in use (see close-up view in FIG. 1D). The one or more pockets 120 each contain (*via* an opening or insert 125) a respective heat pack 130 for a sustained, targeted, and controlled heat transfer to the user (see FIG. 1E); and the one or more pockets 120 comprise a built-in heat zone on the inner radial surface of the heated travel neck pillow 100. Features of the pocket 120, the opening or insert 125, and the heat pack 130 are described in greater detail below.

The pillow 100 comprises a U-shaped pillow having the built-in heat zone on the inner middle surface (on the base 114) and the inner side surfaces (on leg portion 110 and/or 112) of the U-shaped pillow.

In FIG. 1A, the pillow features a soft, plush material on three sides and a flexible spandex bottom so internal micro-beads can shape to and support the neck of a user. Preferably the material of the heated travel pillow comprises the following composition: Outer Shell: 94% Polyester, 6% Spandex; and Filling: 100% Polystyrene Foam Beads.

In FIGs. 1B and 1C; the heated travel neck pillow 100 is comprised of a supportive memory foam core and a soft, plush material on all sides (*i.e.,* a heated travel memory foam neck pillow). Preferably, the material of the heated travel memory foam pillow comprises the following composition: Outer Shell: 99% Polyester, 1% Spandex; and Filling: 100% Polyurethane Foam.

According to the embodiment, the entire heated travel neck pillow 100 in which the heat pack is incorporated is not heated. For example, a designated 'heat zone' comprises an area that comes into contact with the back of the user's neck. This 'heat zone' features a sewn-in, or otherwise attached (e.g., glued on), pocket or pouch (e.g., pocket 120), which may be fastened at one or more open ends 125 by Velcro®, zipper, spandex, elastic, snaps, buttons, or the like. The user activates the heat pack 130 by exposing it to air, and then places it within the pocket 120 on the item. The activated heat pack 130 warms the 'heat zone' to provide soothing heat for a sustained period of time in a targeted area during the user's trip, when relaxing at home, or whenever used by the user.

In the embodiment, the pocket 120 is comprised of a lightweigh, permeable breathable, flexible, stretchable material. Preferably, the pocket is comprised of a spandex material or the like that is lightweight, permeable, breathable, flexible, and stretchable. These properties of the material (e.g., spandex) provide superior contact with the user, while allowing for an optimal amount of heat to permeate from the pocket to the user to optimize warmth. Moreover, an optimal air flow is achieved due to the permeability of the pocket.

The geometry of the pocket is such that the heat pack fits within while having sufficient breathing room and tautness to allow for maximum permeability. Thus, the material of the pocket and the geometry of the pocket provide a 'heat zone' with a sustained, targeted, and controlled heat transfer to a specific area of the user's body.

Vertical and/or horizontal ribbing may be provided within the pocket to enhance air flow and in particular enhance two-way air flow. The ribbing may be provided in one or more portions of the pocket and on one or both internal sides of the pocket.

The flexible and stretchable material allows for ease of insertion of a new heat pack, ease of removal of an exhausted heat pack, support to keep the activated heat pack in place to target the generated heat, and transfer the heat generated to an area on the user's body providing sustained, controlled warmth. The flexible/stretchable material may be expanded by, for example, a user's hand to allow for easy insertion of the heat pack, may return to its original shape or position to help hold the heat pack in place during use, and then expand again to facilitate removal of the exhausted heat pack.

As the heat packs are generally designed to be single use and disposable, the heat pack is easily removed from the pillow's 'heat zone' pocket, so a new one can replace an exhausted pack at a later time, such as the return leg of the user's trip. With this in mind, for sales considerations, the pillow may be bundled with two or more heat packs for the customer to use. Refill packs may also be merchandised as a separate complementary item.

The built-in heat zone comprises a pocket with a heat pack disposed therein. The built-in heat zone may comprise more pockets with one or more heat packs disposed within each of the pockets. The built-in heat zone may comprise a plurality of pockets with more heat packs disposed within each pocket.

In a preferred embodiment, the heat packs are sized and shaped to fit within a 3" x 5" (7.6 cm x 12.7 cm) heat zone (*e.g*., pocket) without bunching or folding; in this particular embodiment, the size of the heat pack is 2.75" (7 cm) H x 4.50" (11.4 cm) W. In another preferred embodiment, the heat pack is roughly 2.75" (7 cm) H x 3.75" (9.5 cm) W, with the pocket accordingly sized to receive and contain the heat pack. In other preferred embodiments, if other sized heat packs are utilized, the pocket is sized with a specific alignment and geometry to the heat pack to provide for sufficient breathing room and tautness for maximum permeability.

## Claims

1. A pillow (100) comprising:
- a U-shaped pillow body comprising two legs (110, 112) and a base (114) therebetween, configured to receive and support a neck of a user;
- a pocket (120) formed on an inner radial portion of the U-shaped pillow body, wherein the inner radial portion is a portion at least partially adjacent to the back of the neck of the user when in use, wherein the pocket (120) is comprised of a lightweight, permeable, breathable, flexible, stretchable material, and is adjacent to the back of the neck of the user when in use; and
- a heat pack (130) contained in the pocket for a sustained, targeted, and controlled heat transfer to the user, wherein the heat pack is an air-activated, disposable heat pack comprising a soft, lightweight, air-permeable pouch.

2. A pillow according to Claim 1, wherein the U-shaped pillow body comprises one or more of foam, visco-elastic memory foam, polyurethane foam, cotton, polyester, polyester plush, spandex, micro-beads, and Polystyrene foam beads.

3. A pillow according to any of Claims 1 to 2, wherein the pocket (120) is comprised of one or more of spandex, cotton, and polyester.

4. A pillow according to any of Claims 1 to 3, wherein the heat pack (130) comprises one or more of iron powder, sawdust, activated carbon, salt, water, and vermiculite to generate heat *via* an oxidation reaction.

5. A pillow according to any of Claims 1 to 4, wherein the pocket (120) comprises one or more open ends (125) for removably receiving and containing the respective heat pack (130).

6. A pillow according to Claim 5, wherein the pocket (120) further comprises a fastener at the one or more open ends (125) for securing the respective heat pack (130) therein, the fastener comprising one or more of a flap, spandex, Velcro®, a snap, a button, and a zipper.

7. A pillow according to any of Claims 1 to 6, wherein the pocket (120) is configured to simultaneously and removably receive and contain a plurality of heat packs (130).

8. A pillow according to claim 1, wherein the pocket (120) is sized and shaped to provide air flow to and from the heat pack (130) contained therein.

9. A pillow according to claim 1, wherein the pocket (120) further comprises ribbing on at least one internal portion of the pocket, for enhancing air flow of the heat pack (130) within the pocket.

10. A pillow according to claim 1, wherein the U-shaped pillow body comprises:
an outer covering comprising (i) a flexible spandex bottom portion and (ii) soft, plush material on side and top portions of the U-shaped pillow body; and
a plurality of micro-beads internal to the outer covering, wherein the micro-beads shape to and support the neck of the user.

11. A pillow according to claim 1, wherein the U-shaped pillow body comprises:
an outer covering comprising a soft, plush material; and
a supportive memory foam core internal to the outer covering.

12. A pillow according to claim 1, further comprising:
one or more supplemental pockets formed on the inner radial portion of the U-shaped pillow body, wherein the one or more supplemental pockets are adjacent to a portion of the neck of the user when in use; and
wherein each supplemental pocket contains a heat pack for a sustained, targeted, and controlled heat transfer to the user.

## Patentansprüche

1. Kissen (100), Folgendes umfassend:
- einen U-förmigen Kissenkörper, umfassend zwei Beine (110, 112) und ein Basisteil (114) dazwischen, dafür konfiguriert, einen Nacken eines Benutzers aufzunehmen und zu stützen;
- eine Tasche (120), die auf einem inneren radialen Abschnitt des U-förmigen Kissenkörpers ausgebildet ist, wobei der innere radiale Abschnitt ein Abschnitt ist, der im Gebrauch wenigstens teilweise an den Nacken des Benutzers angrenzt, wobei die Tasche (120) aus einem leichtgewichtigen, durchlässigen, atmungsaktiven, flexiblen, dehnbaren Material besteht und im Gebrauch an den Nacken des Benutzers angrenzt; und
- eine in der Tasche enthaltene Wärmepackung (130) für eine anhaltende, gezielte und gesteuerte Wärmeübertragung an den Benutzer, wobei die Wärmepackung eine atmungsaktive Einweg-Wärmepackung ist, die einen weichen, leichtgewichtigen, durchlässigen Beutel umfasst.

2. Kissen nach Anspruch 1, wobei der U-förmige Kissenkörper eines oder mehrere umfasst von Schaum, viskoelastischem Speicherschaum, Polyurethanschaum, Baumwolle, Polyester, Polyesterplüsch, Spandex, Mikroperlen und Polystyrenschaumperlen.

3. Kissen nach einem der Ansprüche 1 bis 2, wobei die Tasche (120) aus einem oder mehreren von Spandex, Baumwolle und Polyester besteht.

4. Kissen nach einem der Ansprüche 1 bis 3, wobei die Wärmepackung (130) eines oder mehrere umfasst von Eisenpulver, Sägemehl, Aktivkohle, Salz, Wasser und Vermiculit, um mit einer Oxidationsreaktion Wärme zu erzeugen.

5. Kissen nach einem der Ansprüche 1 bis 4, wobei die Tasche (120) ein oder mehrere offene Enden (125) zum entfernbaren Aufnehmen und Enthalten der entsprechende Wärmepackung (130) umfasst.

6. Kissen nach Anspruch 5, wobei die Tasche (120) weiter ein Befestigungselement an dem einen oder den mehreren offenen Enden (125) umfasst, um die entsprechende Wärmepackung (130) darin zu sichern, wobei das Befestigungselement eines oder mehrere umfasst aus einer Klappe, Spandex, Klettverschluss, einem Druckknopf, einem Knopf und einem Reißverschluss.

7. Kissen nach einem der Ansprüche 1 bis 6, wobei die Tasche (120) dafür konfiguriert ist, um gleichzeitig und entfernbar eine Vielzahl von Wärmepackungen (130) aufzunehmen und zu enthalten.

8. Kissen nach Anspruch 1, wobei die Tasche (120) dimensioniert und geformt ist, um Luftstrom zu und aus der darin enthaltenen Wärmepackung (130) bereitzustellen.

9. Kissen nach Anspruch 1, wobei die Tasche (120) weiter Rippung an wenigstens einem inneren Abschnitt der Tasche umfasst, um den Luftstrom der Wärmepackung (130) innerhalb der Tasche zu fördern.

10. Kissen nach Anspruch 1, wobei der U-förmige Kissenkörper Folgendes umfasst:
einen äußeren Bezug, umfassend (i) einen flexiblen Spandexbodenabschnitt und (ii) weiches, plüschiges Material auf den seitlichen und oberen Abschnitten des U-förmigen Kissenkörpers; und
eine Vielzahl von Mikroperlen innerhalb des äußeren Bezugs, wobei die Mikroperlen die Form des Nackens des Benutzers annehmen und diesen stützen.

11. Kissen nach Anspruch 1, wobei der U-förmige Kissenkörper Folgendes umfasst:
einen äußeren Bezug, umfassend ein weiches, plüschiges Material; und
einen stützenden Speicherschaumkern innerhalb des äußeren Bezugs.

12. Kissen nach Anspruch 1, weiter umfassend:
eine oder mehrere auf dem inneren radialen Abschnitt des U-förmigen Kissenkörpers ausgebildete zusätzliche Taschen, wobei die einen oder mehreren zusätzlichen Taschen im Gebrauch an einen Abschnitt des Nackens des Benutzers angrenzen; und
wobei jede zusätzliche Tasche eine Wärmepackung für eine anhaltende, gezielte und gesteuerte Wärmeübertragung an den Benutzer enthält.

## Revendications

1. Oreiller (100), comprenant :
- un corps d'oreiller en U, doté de deux montants (110, 112) et d'une base (114) entre eux, configurés pour recevoir et soutenir le col d'un utilisateur ;
- une poche (120) pratiquée dans une partie radiale intérieure d'un corps d'oreiller en U, la partie radiale intérieure étant une partie au moins partiellement adjacente, en cours d'usage, à la nuque de l'utilisateur, la poche (120) étant composée d'un tissu léger, perméable, respirant, flexible, et extensible, et étant adjacente à la nuque de l'utilisateur en cours d'usage ; et
- un pack de chaleur (130) contenu dans la poche, pour assurer un transfert constant, ciblé et contrôlé de la chaleur à l'utilisateur, le pack de chaleur étant un pack de chaleur à usage unique activé par l'air, comprenant une pochette souple, légère et perméable à l'air.

2. Oreiller selon la revendication 1, le corps d'oreiller en U comprenant un ou plusieurs des suivants : mousse, mousse à mémoire viscoélastique, mousse de polyuréthane, coton, polyester, peluche de polyester, spandex, microbilles et billes de mousse de polystyrène.

3. Oreiller selon une quelconque des revendications 1 à 2, la poche (120) étant composée d'un ou plusieurs des suivants : spandex, coton et polyester.

4. Oreiller selon une quelconque des revendications 1 à 3, le pack de chaleur (130) comprenant un ou plusieurs des suivants : poudre de fer, sciure de bois, charbon actif, sel, eau, et vermiculite, pour la production de chaleur par le biais d'une réaction d'oxydation.

5. Oreiller selon une quelconque des revendications 1 à 4, la poche (120) comprenant une ou plusieurs extrémités ouvertes (125) pour recevoir et contenir de façon amovible le pack de chaleur (130) respectif.

6. Oreiller selon la revendication 5, la poche (120) comprenant en outre un dispositif de fermeture à la ou plusieurs des extrémités ouvertes (125) pour la fixation dans celle-ci du pack de chaleur(130) respectif, le dispositif de fermeture comprenant un ou plusieurs des suivants : rabat, spandex, Velcro®, bouton pression, bouton et fermeture éclair.

7. Oreiller selon une quelconque des revendications 1 à 6, la poche (120) étant configurée pour recevoir et contenir, simultanément et de façon amovible, une pluralité de packs de chaleur (130).

8. Oreiller selon la revendication 1, la poche (120) étant dimensionnée et façonnée de façon à permettre un écoulement d'air vers le pack de chaleur (130) qu'elle contient, et en provenance de celui-ci.

9. Oreiller selon la revendication 1, la poche (120) comprenant en outre des nervures sur au moins une de ses parties internes de la poche, afin de renforcer l'écoulement d'air du pack de chaleur (130) dans la poche.

10. Oreiller selon la revendication 1, le corps de l'oreiller en U comprenant :
un revêtement extérieur composé (i) d'une partie inférieure souple en spandex, et (ii) d'un tissu souple et feutré sur les parties latérales et supérieures du corps de l'oreiller en U ; et
une pluralité de microbilles intérieures au revêtement extérieur, les microbilles s'adaptant à la forme du cou de l'utilisateur et le supportant.

11. Oreiller selon la revendication 1, le corps de l'oreiller en U comprenant :
un revêtement extérieur comprenant un tissu souple et feutré ; et
un noyau de mousse de soutien à mémoire à l'intérieur du revêtement extérieur.

12. Oreiller selon la revendication 1, comprenant également :
une ou plusieurs poches supplémentaires formées sur la partie radiale intérieure du corps de l'oreiller en U, la ou plusieurs poches supplémentaires étant adjacentes, en cours d'usage, à une partie du cou de l'utilisateur ; et
chaque poche supplémentaire contenant un pack de chaleur pour le transfert continu, ciblé et contrôlé de la chaleur à l'utilisateur.
